# DEMANDE DE BREVET EUROPEEN

(11) **EP 0 925 764 A1**
(43) Date de publication de la demande: **30.06.1999**
(21) Numéro de dépôt: 98830755.9
(22) Date de dépôt: 15.12.1998
(51) Int. Cl.: A61F 2/26

(54) **Prothèse pénienne de silicone semi-rigide dynamique anti érosion**

(30) Priorité: 19.12.1997 IT MI972805
(71) Demandeur: Grasso, Marco, 20129 Milano (IT); Scalisi, Giuseppe, 20156 Milano (IT)
(72) Inventeur: Grasso Marco, 20129 Milano (IT); Scalisi Giuseppe, 20156 Milano (IT)

(57) **Abrégé**

Prothèse pénienne à installer dans les corps caverneux composée de deux barrettes en silicone Medical Grade assemblant harmonieusement trois zones aux caractéristiques différentes : La zone centrale (fig.1point-B) cylindrique a un diamètre et une rigidité variables pour répondre aux différentes exigences cliniques et garantir la pénétration. La zone distale (fig.1point-C), d'un silicone plus souple, réduit l'érosion des tissus, nouveauté qui en fait une prothèse anti érosion. La zone proximale (fig.1point-C), d'un diamètre inférieur aux 3 mm, constituée d'une succession de segments, confère la particularité de faciliter l'introduction dans les corps caverneux, même en présence de fibrose. La zone centrale, par sa dureté et le frottement avec les corps caverneux, confère à la verge un effet de semi-rigidité.La zone proximale, elle, grâce à son diamètre plus petit est plus flexible et, rencontrant moins de frottement, plus libre de se plier garantissant ainsi à la prothèse une dissimulation facile.

## Description

L'implantation d'une prothèse endocaverneuse se présente souvent comme la meilleure solution dans l'approche thérapeutique à l'impuissance organique.

Les prothèses actuellement utilisées peuvent être divisées en trois types :
1) Prothèses hydrauliques: composées en général d'un corps creux gonflable avec un liquide poussé par pression par un système plus ou moins complexe.
   Ces types de prothèses qui respectent l'alternance physiologique procurent des avantages mais, étant des systèmes munis de soupapes et de pompe, ils sont soumis à une usure avec ruptures possibles et donc nécessité de réintervention; en outre il présentent l'inconvénient que le porteur doit effectuer des opérations manuelles qui ne sont pas toujours acceptées par manque d'acceptation ou à cause de la difficulté à les effectuer correctement.
2) Prothèses rigides, mécaniques, malléables; en général elles contiennent à l'intérieur un corps métallique, en argent ou autre matériau, structuré en forme de tresse ou d'autre façon, pour permettre une certaine possibilité de changement de position.
   Ces prothèses procurent à la verge une rigidité qui permettent une bonne pénétration; mais en dehors de cet avantage, elles deviennent fastidieuses et inconfortables pour ceux qui les portent, à cause de leur manque de flexibilité; problèmes que même les mesures plus ou moins réussies de conférer une rigidité différente à la partie proximale par rapport à la partie distale, de façon à permettre une certaine flexibilité, n'ont pas réussi à résoudre.
   Les prothèses rigides, de leur nature, peuvent être sujettes avec le temps à d'éventuelles ruptures ou raidissements des points articulés, facteurs qui portent à la nécessité d'une nouvelle intervention; en outre, a cause de leur rigidité justement, ce sont des prothèses qui peuvent avoir une incidence d'érosion plus élevée que d'autres.
3) Prothèses souples en silicone: ce sont des cylindres de silicone plus ou moins souples qui, tout en garantissant une consistance suffisante pour permettre la pénétration, sont confortables car faciles à dissimuler. Résistantes à la traction ou à l'écrasement, excluant les traumatismes, elles ne sont pas sujettes à ruptures; grâce à ces caractéristiques, les prothèses souples en silicone peuvent être considérées comme les plus sûres et confortables par rapport aux autres types.

Les prothèses souples que l'on trouve dans le commerce présentent cependant des inconvénients: elles peuvent provoquer des érosion, quoique proportionnellement moins que les prothèses rigides; elles peuvent en outre présenter certaines difficultés d'insertion dans la position crurale des corps caverneux, à l'intérieur desquels le glissement d'un cylindre souple à l'extrémité tronquée présente une difficulté considérable, surtout en présence de fibrose.

Tandis que pour les prothèses dans le commerce aucune mesure n'est utilisée pour réduire les cas d'érosion, les solutions actuellement utilisées pour rendre l'insertion plus facile sont l'arrondissage, en cours d'opération, de la partie coupée, ou la greffe d'un capuchon de quelques centimètres avec une partie terminale conique, solution qui peut créer des problèmes car, en l'insérant sur la prothèse, on provoque une augmentation du diamètre de 2 mm qui rend encore plus difficile la passage dans les corps caverneux; en outre, elle ne répond pas à un critère important pour une prothèse, c'est à dire d'être un corps unique indéformable. Une autre solution possible serait l'installation de prothèses de longueur variable aux extrémités coniques, ce qui comporte la nécessité d'avoir pour chaque intervention la disponibilité de nombreuses prothèses afin de pouvoir choisir celle dont la longueur est adéquate pour le patient.

Quoi qu'il en soit, l'insertion de la prothèse dans la zone crurale est un problème qui, quelle que soit la dilatation des corps caverneux, est dans certains cas plutôt difficile à résoudre, tandis que dans d'autres cas on est obligé de choisir l'utilisation d'un prothèse d'un diamètre inférieur au diamètre optimal.

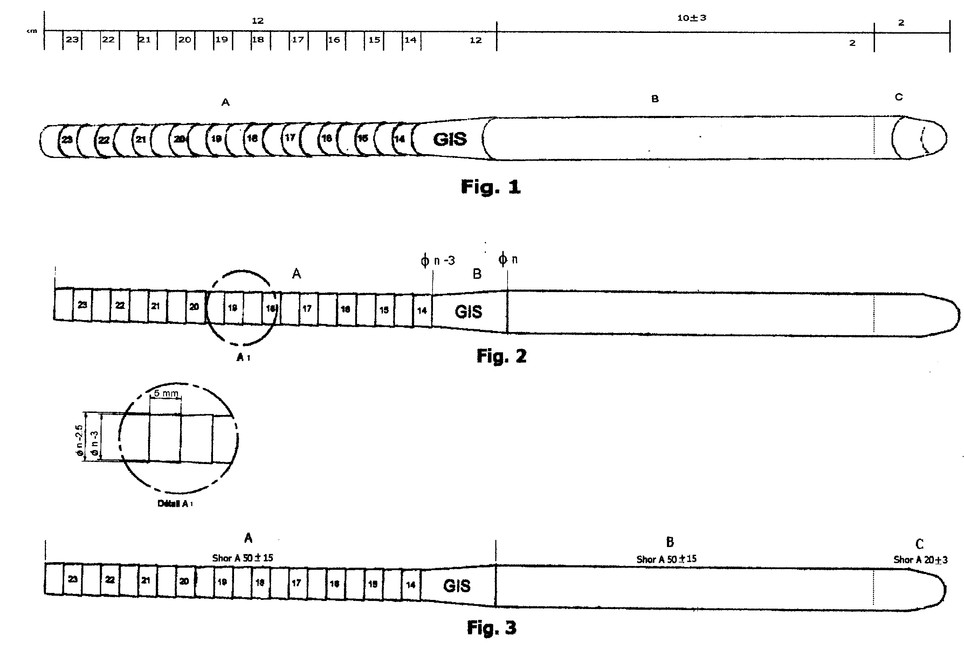

La prothèse pénienne de silicone bio-compatible, objet de ce brevet, est composée de deux cylindres blancs à installer, plus un autre coloré pour la détermination de la longueur;
Chacun des élastomères assemble de façon harmonieuse trois zones aux caractéristiques différentes spécifiées dans la description. Les dessins annexés en illustrent la disposition et la forme :
- □: la figure 1 présente une barrette de l'implantation de la prothèse
- □: la figure 2 (point A et point A1 agrandi) visualisent les détails de la zone proximale
- □: la figure 3 montre la délimitation par dureté des trois zones

La zone proximale, longue de 12 cm FIG.1 (point A), présente un segment de 2 cm FIG.2 (point B) en cône tronqué avec une différence de 3 mm entre les deux diamètres, et 20 autres de 5 mm FIG.2 (point A et détail agrandi A1) de forme légèrement conique avec une différence de 5 mm entre les deux diamètres.

La zone centrale a une longueur qui, selon les modèles, peut varier de 7 à 13 cm fig.1 (point B), et a une forme cylindrique dont le diamètre peut varier de 8 à 15 mm. La zone distale de 2 cm fig.1 (point C) est de forme cylindrique, comme la zone centrale, mais avec une extrémité arrondie.

La longueur, le diamètre et la rigidité de la zone centrale, fig.3 (point B), variables selon les modèles, répondent aux différentes exigences cliniques et assurent une bonne pénétration.

La structure particulière de la zone proximale caractérise les prothèses péniennes objet de ce brevet, et présente les avantages suivants :
- •: Il s'agit d'une prédisposition pour l'insertion facilitée dans les corps caverneux.
- •: Elle rend la prothèse plus dynamique et mobile, en facilitant son introduction dans les corps caverneux; car avec la série de segments à section réduite de 3 mm, par rapport à la zone centrale, (FIG. 2 point A) même en présence de fibrose, elle est très facile à introduire et à en forcer le passage.
- •: Elle facilite la mesure et la coupe à la longueur adéquate à chaque cas spécifique
- •: Elle rend la zone proximale plus flexible que la centrale, grâce à la différence des deux diamètres, mais aussi parce que la zone proximale rencontre moins de frottement avec les parois des corps caverneux par rapport au reste de la prothèse qui, en occupant presque toute la lumière, présente plus de résistance aux mouvements et acquiert une certaine rigidité.

Grâce à ces caractéristiques, on a donné à la prothèse objet de ce brevet le nom de PROTHESE PENIENNE SSD (de SILICONE SEMI-RIGIDE DYNAMIQUE).

La flexibilité différente des deux parties peut être accentuée en différenciant la dureté entre les deux zones; celle de la zone centrale (FIG. 3 point B) peut osciller entre 35 et 65 shore A et en fonction de cela on peut obtenir une meilleure pénétration, tandis que celle de la zone proximale (FIG. 3 point A), reste, dans les modèles les plus rigides, à des niveaux plus bas afin de ne pas perdre la caractéristique de prothèse confortable; tous les modèles permettent de toute façon une bonne pénétration.

L'extrémité distale de 2 cm (fig. 3 point C), avec une partie terminale toujours arrondie, est en silicone le plus souple possible, avec une dureté inférieure à 24 shore A; elle remplit donc une fonction de coussinet amortisseur (FIG. 3 point C) qui peut limiter les érosions éventuelles des tissus; les prothèses objet de ce brevet, produites selon ces caractéristiques, peuvent être définies ANTI EROSION.

Cette innovation, nécessaire pour les modèles au corps central plus dur et utile pour les autres, est une mesure qui fait de la PROTHESE PENIENNE de SILICONE SEMI-RIGIDE DYNAMIQUE la seule avec des caractéristiques ANTI EROSION;
L'assemblage harmonieux de ces trois zones présentant les caractéristiques spécifiées représente l'innovation qui fait de la PROTHESE PENIENNE SSDA (de SILICONE SEMI-RIGIDE DYNAMIQUE ANTI EROSION) une prothèse différente des autres en vente dans le commerce.

## Revendications

1. Prothèse pénienne, caractérisée par le fait que les élastomères de silicone Medical Grade bio-compatible, deux blancs à insérer dans les corps caverneux, et un coloré pour la détermination de la mesure adéquate au cas examiné, assemblent de manière harmonieuse trois zones dont le diamètre, la forme, la dureté et l'élasticité du silicone sont différents fig. 2 et fig.3

2. Prothèse comme présenté par la revendication 1 caractérisée par le fait que la zone proximale, longue de 12 cm, présente un segment de 2 cm FIG. 2 (point B) en cône tronqué avec une différence de 3 mm entre les deux diamètres et 20 autres de 5 mm chacun FIG. 2 (point A et détail A1) de forme légèrement conique avec une différence de 0,5 mm entre les deux diamètres.

3. Prothèse comme présenté par les revendications 1 et 2 caractérisée par le fait que la mesure de la longueur adéquate au cas examiné est facilitée par la succession des segments coniques fig.2 (point A).

4. Prothèse comme présenté par les revendications 1, 2 et 3 caractérisée par le fait que, en cours d'opération, une simple coupe, dans la partie la plus étroite du segment fig.2 (point A1) suffit pour délimiter la partie proximale de la prothèse, avec partie terminale adéquate pour faciliter l'introduction dans les corps caverneux.

5. Prothèse comme présenté par les revendications 1,2,3 et 4 caractérisée par le fait que le segment en forme de cône tronqué de 2 cm de la zone proximale FIG.2 (point B) fait office de dilatateur des corps caverneux pour le passage de la partie restante de la prothèse;

6. Prothèse comme présenté par les revendications 1,2,3,4 et 5 caractérisée par le fait que la zone proximale, grâce à son diamètre plus petit, est plus flexible et plus libre de se plier, par rapport au reste de la prothèse qui, plus rigide et empêchée par le frottement avec le corps caverneux, confère un effet de semi-rigidité à la verge.

7. Prothèse comme présenté par les revendications 1,2,3,4,5 et 6 caractérisée par le fait que pour certains modèles la différence d'élasticité entre la zone proximale et la zone centrale peut être accentué par une plus grande dureté du silicone de la zone centrale FIG.3 (point B) qui acquiert une plus grande rigidité, ou par une dureté inférieure du silicone de la zone proximale FIG.3 (point A) qui acquiert une plus grande flexibilité, les duretés peuvent varier de 35 à 65 shore A.

8. Prothèse comme présenté par la revendication 7 caractérisée par le fait que la zone distale de 2 cm fig.3 (point C) est formée de silicone le plus souple possible avec une dureté inférieure à 24 shore A.

9. Prothèse comme présente par les revendications 7 et 8 caractérisée par le fait que, grâce à l'utilisation d'un coussinet plus souple dans la partie distale, il s'agit d'une prothèse pénienne offrant une précaution anti érosion.

10. Prothèse comme présenté par les revendications précédentes caractérisée par le fait qu'il s'agit de la seule PROTHESE PENIENNE de SILICONE SEMI-RIGIDE DYNAMIQUE ANTI EROSION.
